# EUROPÄISCHE PATENTANMELDUNG

(11) **EP 2 803 348 A1**
(43) Veröffentlichungstag der Anmeldung: **19.11.2014**
(21) Anmeldenummer: 13167786.6
(22) Anmeldetag: 15.05.2013
(51) Int. Cl.: A61J 1/20, A61M 5/178, A61K 9/08, A61J 1/10, A61M 1/00, A61J 3/00

(54) **Verfahren zur Befüllung von Spritzen für Dosierpumpen**

(71) Anmelder: hameln rds gmbh, 31789 Hameln (DE)
(72) Erfinder: Dewald, Mathias, 31787 Hameln (DE); Wegener, Olaf, 31848 Bad Münder (DE); Ewald, Gerhard, 34212 Melsungen (DE)

(57) **Zusammenfassung**

Die vorliegende Erfindung betrifft gebrauchsfertige Lösungen parenteral zu verabreichender Medikamente in kollabierbaren, luft- und feuchtigkeitsundurchlässigen Behältern. Erfindungsgemäß sind die Behälter so konzipiert, dass die parenteralen Lösungen direkt ohne weitere Hilfsmittel zur Verabreichung in Spritzen für Dosierpumpen (50 ml) aufgezogen werden können.

## Beschreibung

### Gebiet der Erfindung

Die vorliegende Erfindung betrifft gebrauchsfertige Lösungen parenteral zu verabreichender Medikamente in kollabierbaren, luft- und feuchtigkeitsundurchlässigen Behältern. Erfindungsgemäß sind die Behälter so konzipiert, dass die parenteralen Lösungen direkt ohne weitere Hilfsmittel zur Verabreichung in Spritzen für Dosierpumpen (50 ml) aufgezogen werden können.

### STAND DER TECHNIK

Hypnotika, Schmerzmittel, Cytostatika, aber auch andere parenteral zu verabreichende Wirkstoffe werden im intensivmedizinischen Bereich in großer Zahl über Dosierpumpen infundiert. Hierbei handelt es sich um Spritzenpumpen, die schrittweise auch kleinste Mengen von Flüssigkeit abgeben und so eine möglichst konstante Wirkstoffkonzentration im Blut erzielen. Für den Gebrauch von Dosierpumpen werden in der Regel großvolumige Spritzen (generell 50 ml) eingesetzt.

Die Vorbereitung der Spritzen ist für das medizinische Personal mit hohem Aufwand und Risiken verbunden.

Parenteral zu verabreichende Medikamente werden standardmäßig nach Produktion in Glasampullen abgefüllt, die eine oder mehrere Dosen aufnehmen können. Zur Verabreichung der Medikamente z.B. durch Spritzenpumpen sind mehrere Handlungsschritte notwendig.

In der Praxis gängig sind Glasampullen für Einmaldosen, die mittels einer speziellen Ampullensäge zu öffnen sind oder mit einer Sollbruchstelle versehen werden. Nach Öffnen der Ampulle wird der Inhalt vom medizinischen Personal über eine Injektionskanüle direkt in die 50 ml Spritze aufgezogen oder falls nötig in einen weiteren Behälter zum Verdünnen gefüllt und anschließend in die Spritze aufgezogen. Hierbei muss etwas Lösung in der Ampulle verbleiben, um das Ansaugen von Luft zu verhindern. Ein Verschließen der Restlösung bzw. ein Weiterverwenden ist nicht möglich.

Selbst wenn die Lösung direkt in die zur Verabreichung geeignete Spritze aufgezogen wurde, kann die eingesetzte Kanüle nicht weiter verwendet werden, da sie unsteriler Luft ausgesetzt wurde. Eine weitere Injektionskanüle bzw. ein Infusionsbesteck werden benötigt. Jede weitere verwendete Kanüle bedeutet einen Zeit- und Kostenaufwand. Des Weiteren können beim Aufbrechen der Ampulle Glassplitter entstehen, die Personal und Patient gefährden könnten.

Alternativ werden parenterale Produkte als Einmal- oder Multidosen in sogenannte Stechampullen, auch Vial genannt, abgefüllt. Vials sind in der Regel ebenfalls aus Glas und mit einem Gummistopfen verschlossen. Weiterhin wird der Stopfen durch eine Plastik- oder Metallkappe geschützt. Zur Verabreichung der Injektionslösung muss zunächst die Kappe entfernt und der Stopfen mit einer Kanüle durchstochen werden. Im Vorfeld wird der Stopfen häufig mit einem Desinfektionsmittel gesäubert. Das Durchstechen des Stopfens birgt das Risiko einer Verunreinigung der Injektionslösung mit Gummipartikeln.

Zudem ist die Handhabung aufwendig und kann zur Kontamination des Produktes mit unsteriler Luft führen. So wird oft über die Spritze zunächst Luft angesaugt und in das Vial injiziert, um einen Überdruck zu erzeugen. Ohne den Überdruck ist eine Extraktion in die Spritze schwierig, da dann im Vial ein Unterdruck entsteht. Gerade das Befüllen großvolumiger Spritzen ist mit einem beträchtlichen Kraftaufwand verbunden, der mit mehr als 100 N angegeben wird. Wird nicht absichtlich vor Extraktion Luft in das Vial gespritzt, kann es dennoch zu einer Kontamination mit Umgebungsluft kommen, da gerade beim Aufziehen großer Volumen ein nicht unbeträchtlicher Unterdruck entsteht, der beispielsweise zum Druckausgleich am Spritzenkolben führt. Kontamination mit Umgebungsluft birgt ein hohes Infektionspotential für den Patienten. Der hohe Kraftaufwand sowie der Einsatz einer zusätzlichen Kanüle bergen zudem Verletzungsrisiken für das medizinische Personal. Gerade im intensivmedizinischen Bereich werden oft hoch potente Wirkstoffe wie Cytostatika über Spritzenpumpen verabreicht, was eine große Gefahr für das Personal darstellt.

Das Benzodiazepin Midazolam wird in der Intensivmedizin zur Sedierung als Dauerinfusion über eine Spritzenpumpe verabreicht. Für diesen Zweck wird es derzeit als fertige 50 ml Lösung in Stechampullen vertrieben.

Glyceroltrinitrat (GTN) wird in der Intensivmedizin zur Behandlung akuter Herzinfarkte ebenfalls kontinuierlich über eine Dosierpumpe verabreicht. Auch GTN ist derzeit als fertige 50 ml Lösung in Stechampullen auf dem Markt.

Auch die Muskelrelaxanzien Atracurium bzw. Cis-Atracurium werden in der Intensivmedizin als Dauerinfusion über Spritzenpumpen eingesetzt. Atracurium und Cis-Atracurium sind als 2.5 und 5 ml Ampullen erhältlich. Zur Vorbereitung einer Dauerinfusion muss entsprechend eine Verdünnung auf 50 ml erfolgen.

Verschiedene Lösungsansätze sind in der Literatur beschrieben, um Kosten, Aufwand und Risiken der Vorbereitung von Infusions- und Injektionslösungen, im Besonderen das Aufziehen der Parenteralia in Injektionsspritzen, zu minimieren.

Beispielsweise vertreibt die Firma B. Braun Melsungen AG zu diesem Zweck sogenannte Mini-Spikes®. Hierbei handelt es sich um Plastikspikes mit Luer-Lock Anschluss zum direkten Ansetzen der Spritze. Weiterhin verfügt der Spike über einen Belüftungsfilter mit Kontaminationsschutz.

Jeder Spike zur Entnahme erhöht allerdings die Kosten des Injektions- bzw. Infusionsvorgangs. Zudem wird das Handling zeitaufwendig. Der Einsatz jeder zusätzlichen Kanüle birgt zudem wieder ein Verletzungs- und Kontaminationsrisiko. Bei hochpotenten Wirkstoffen außerdem somit ein hohes Gesundheitsrisiko für das medizinische Personal.

Auch eine Abfüllung direkt in eine Spritze ist technisch möglich. Eine Spritze weist neben hohen Kosten jedoch den Nachteil auf, dass sie aus mehreren Teilen unterschiedlicher Materialien aufgebaut ist, was die Wahrscheinlichkeit einer Unverträglichkeit des Wirkstoffs oder der Hilfsstoffe mit den Materialien erhöht und so die Haltbarkeit und Lagerung einschränkt. Weiterhin müssen Spritzenkolben- und zylinder meist mit Silikonen beschichtet werden, um eine Funktionsfähigkeit während der ganzen Laufzeit zu gewährleisten. Auch dies erhöht wiederum das Risiko einer Unverträglichkeit des Medikaments mit dem Primärpackmittel.

Eine direkte Abfüllung in Spritzen eignet sich für die Bereitstellung der großvolumigen Intensivmedikamente zum Einsatz in Dosierpumpen auch deshalb nicht, da Spritzenpumpen verschiedener Hersteller und somit verschiedener Bauarten eingesetzt werden. Jede Spritzenpumpe hat andere Anforderungen an die einzusetzenden Spritzen.

Weitere Lösungsansätze zur Vermeidung der Probleme beim Transfer einer Injektions- oder Infusionslösung vom Lagerbehälter in die Verabreichungsvorrichtung werden im Stand der Technik beschrieben. Vorgestellt sind Ampullen oder Vials, die direkt mit einer Injektionskanüle oder einem Spritzenzylinder verbunden werden können und so ohne Einsatz einer Zwischenkanüle transferiert oder verabreicht werden könnten. Beispielsweise werden Entwicklungen zu thermoplastischen Behältnissen für parenterale Einzeldosen beschrieben.

FR-A-1 566 283 beschreibt ein kollabierbares Behältnis, welches direkt als Einmalspritze genutzt werden kann. Hierzu ist das Behältnis mit einem männlichen Adapter ausgestattet, an den direkt eine Kanüle angesetzt werden kann. Der quetschbare Behälter ist weiterhin für Medikamente gedacht, die oral verabreicht werden können.

EP-B-1 0 591 156 beschreibt ein Behältnis, das direkt mit einem Spritzenkörper verbunden werden kann, um den Transfer einer Injektionslösung einfacher und sicherer zu gestalten. Das Problem ist dadurch gelöst, dass das Behältnis beispielsweise mit einem Luer Lock Verschluss ausgestattet ist. Des Weiteren sind die Wände des Behälters kollabierbar, was eine Entleerung erleichtert. Das Behältnis ist aus thermoplastischem Material.

US 6,379,342 beschreibt ebenfalls ein flexibles Einmalbehältnis mit einem Luer-Lock Anschluss, welches direkt an Verweilkanülen angeschlossen werden kann. Der direkte Anschluss an einen Venenkatheter ermöglicht die sterile direkte Injektion eines Medikaments wie beispielsweise Heparin. Die zu übertragende Flüssigkeitsmenge liegt im Bereich von 2 cc und weniger oder auch 3 - 5 cc. Auch ein Anschluss an subkutane Kanülen oder Spritzenkörper ist möglich.

Auch EP-B-1 0 088 056 offenbart ein Behältnis für eine Dosiseinheit eines flüssigen Medikaments, das direkt und vollständig in eine Spritze überführt werden kann. Der Behälter ist aus thermoplastischem Material und wurde durch Blow-fill-seal Verfahren gebildet. Die Auslassöffnung des Behälters ist so geformt, dass direkt mit der Spitze einer Spritze eingegriffen und die Lösung auslaufsicher aufgenommen werden kann. Im Besonderen ist der Behälter mit einem Luer-Innenkonus beschrieben. Weiterhin fällt der Behälter beim Entleeren zusammen, was ein Ausleeren erleichtert.

Bedingt durch die Herstellprozesse (Spritzgießen, Blasverfahren oder Extrusion), kommen Materialien zum Einsatz, die im polymerisierten zustand noch verformbar sind. Darunter fallen z.B. Polyethylen (PE) und Polyvinylchlorid (PVC). Diese Materialen haben den Nachteil, dass sie feuchtigkeitsdurchlässig sind, was dazu führt, dass z.B. Wasser durch die thermoplastischen Wände des Behälters nach außen diffundieren kann und über die Laufzeit des Medikamentes zu einem unerwünschten Gehaltsanstieg des Wirkstoffs führt. Auch Sauerstoffpermeabilität des Materials und somit unerwünschte Oxidation können bei sensiblen Wirkstoffen zu Problemen führen. Eine weitere Ausführungsform des Dosiseinheitsbehältnisses ist dadurch beschrieben, dass das Behältnis in einem feuchtigkeitsundurchlässigen Beutel aus einem Laminat aus Metall und Plastik eingeschlossen ist.

Diese Probleme werden beispielsweise auch in US 3,660,194 beschrieben und teilweise gelöst. Hier werden Tuben beschrieben, die kollabierbar und undurchlässig für Flüssigkeiten sind. Auch die hier beschriebenen Tuben sind durch den Herstellungsprozess auf elastische Kunststoffe beschränkt.

Eine Weiterentwicklung wird in WO 2010/135843 der Firma Hoffmann Neopac AG beschrieben. Hier werden gas- und flüssigkeitsundurchlässige, kollabierbare Tuben aus starren oder halbstarren Folien vorgestellt. Die beschriebenen Folien bestehen aus einer Trägerfolie, die auf beiden Oberflächen mit dem gleichen thermoplastischen Material ummantelt ist. Die Trägerfolie kann eine funktionale, schützende Barriere darstellen. Der Tubenkopf soll aus dem gleichen Thermoplasten hergestellt werden wie die Ummantelung der Folie. Als Materialien für die Trägerfolie werden beispielsweise Aluminium, Polyester, Polyamide, aber auch Cylco Olefin Copolymer (COC) oder Ethylen Vinylalkohol Copolymere (EVOH) vorgestellt. Für die Ummantelung kommen Homo- oder Copolymere, namentlich Polyester, Polyamide, COC und bevorzugt Polyethylenterephthalat (PET), PET-P (PET-Homopolymer) oder thermoplastische Polyester Elastomere/thermoplastische Copolyester (TPE-E/TPC) zum Einsatz. Weitere Anmeldungen der Firma Hoffmann Neopac AG beschreiben alternative Tubenausführungen. So beschreibt beispielsweise WO 2012/079180 eine Tube, deren Tubenkopf einen weiblichen Luer-Lock-Anschluss darstellt und so gestaltet ist, dass er zusätzlich den Tubenkopf verschließt. Dies wird durch eine Deckelfolie realisiert, die einen Sicherheitsverschluss darstellt und erst bei Einschrauben der Spritze mit männlichem Luer-Lock geöffnet wird. Dies erlaubt es mit einer Spritze mit männlichem Luer-Lock Anschluss direkt Flüssigkeit aus der Tube aufzuziehen. Die Tube wurde konstruiert um anschließend direkt durch Aufsetzen einer Kanüle auf den männlichen Luer Lock das Medikament durch Injektion zu verabreichen.

Weiterhin wird vorteilshaft das Material der Tube aus PE oder PP ausgewählt, um eine transparente Tube zu erhalten. Die Trägerfolie soll eine Barriere darstellen und wird bevorzugt aus COC, PCTFE oder EVOH dargestellt.

Eine weitere Ausgestaltung einer Tube für die Aufbewahrung flüssiger Arzneimittel wird beispielsweise in EP-B-1 1 883 387 der Firma Vifor beschrieben. Die hier beschriebene Tube verfügt über ein sogenanntes "Knackfroschprinzip", was bewirkt, dass der Behälter durch einfachen Druck mit dem Daumen in einem Zug entleerbar ist. Diese Tuben sind auf ein Volumen von bis zu 5 ml beschränkt.

Weiterhin werden verschiedene Ausführungen von Infusionsbeuteln beschrieben, die ebenfalls zur Aufgabe haben das Handling des Infusionsvorgangs zu vereinfachen und dabei das Risiko einer Verletzung des medizinischen Personals durch Kanülen zu reduzieren. Standardmäßige Infusionsbeutel, die zum Mischen und zur Verabreichung von Parenteralia eingesetzt werden bestehen aus einem Behältnis (Beutel) zur Aufnahme der Lösung, welcher mit einem Zugangsport ausgestattet ist, der es erlaubt über eine Spritze mit aufgesetzter Kanüle flüssige Materialien zuzuführen oder zu entnehmen. US-A-1 2005/0059952 beschreibt beispielsweise eine Weiterentwicklung dieser Technik durch die sowohl Kontaminationsrisiken der Infusionslösung als auch Verletzungsrisiken für das medizinische Personal minimiert werden. Beschrieben wird ein Infusionsbeutel, der flüssiges Material aufnehmen kann ohne dass eine Kanüle eingesetzt werden muss. Hierzu ist der Infusionsbeutel mit einer Zugangskammer ausgestattet, die über einen Port mit Ventil verfügt, der einen Luer-Lock Anschluss darstellt.

Vor dem Hintergrund der oben beschriebenen Nachteile ist es Aufgabe der vorliegenden Erfindung parenteral zu verabreichende Produkte für die Anwendung in Spritzenpumpen so bereitzustellen, dass es möglich ist, die Arzneimittel ohne erhöhten Kraftaufwand und unter Vermeidung von Verletzungsgefahren des medizinischen Personals und Kontamination des Arzneimittels in die benötigten 50 ml Spritzen zu überführen. Die parenteralen Produkte sollen zudem lagerstabil sein.

### Beschreibung der Erfindung

Die erfindungsgemäße Lösung der Aufgabe besteht darin die im intensivmedizinischen Bereich eingesetzten Arzneimittel direkt als gebrauchsfertige großvolumige 50 ml Lösungen bereitzustellen, so dass sie direkt ohne weiteres Verdünnen oder Mischen in Spritzenpumpen (verschiedener Hersteller und Bauarten) eingesetzt werden können. Im Besonderen sieht die Erfindung vor die Arzneimittel in einem kollabierbaren Behältnis bereitzustellen, welches über einen Luer-Lock Anschluss direkt mit der Spritze verbunden werden kann.

Überraschend konnte gezeigt werden, dass verschiedene im intensivmedizinischen Bereich eingesetzte Arzneimittel, als gebrauchsfertige 50 ml Lösungen in großvolumigen, kollabierbaren Tuben stabil gelagert werden können. Beispielhaft werden Lösungen der bevorzugten Medikamente Atracurium, Cis-Atracurium, Glyceryltrinitrat (GTN) und Midazolam in kollabierbaren Tuben gelagert.

Lagerstabil ist ein Produkt danach, wenn es so lange in einem Behältnis chemisch stabil ist, dass eine Lagerhaltung sinnvoll ist. Diese Zeit liegt je nach Wirkstoff zwischen 18 und 36 Monaten. Wenn möglich, so ist die Lagerung bei Raumtemperatur vorzusehen. Einen Hinweis auf die Lagerstabilität geben Daten bei erhöhter Temperatur. Chemisch stabil ist ein Wirkstoff, wenn er in der Lösung nicht in unzumutbarem Maße zu pharmazeutisch unverträglichen Verunreinigungen abgebaut wird. Nähere Angaben zur Stabilität pharmazeutischer Darreichungsformen und zu zumutbaren Mengen von Verunreinigungen geben die Fachliteratur und die Arzneibücher.

Gemäß Arzneibuch enthalten Behälter für injektabile Produkte, die für die Aufnahme von 50 ml vorgesehen sind nicht weniger als 50 ml, was im Sinne dieser Erfindung gleichbedeutend ist mit einem Inhaltsvolumen von 50-60 ml.

Weitere Produkte, die in solchen Behältern denkbar sind, sind Lösungen der Wirkstoffe: Adrenalin, Alfentanil, Alprostadil, Alteplase, Amiodaron, Argatroban, Calciumgluconat, Clonidin, Ceftazidim, Ciclosporin, Clonazepam, Dalteparin, Danaparoid, Dihydralazin, Diltiazem, Dobutamin, Drotrecogin, Enalapril, Enoximon, Epoprostenol, Esketamin, Flunitrazepam, Fentanyl, Furosemid, Heparin, Hydrocortison, Insulin, Ketamin, Lepirudin, Levosimedan, Lidocain, Metamizol, Methylprednisolon, Metoprolol, Magnesiumsulfat, Milrinon, Morphin, Molsidomin, Nifedipin, Nimodipin, Noradrenalin, Orciprenalin, Propafenon, Piritramid, Propofol, Rocuronium, Vasopressin.

Weiterhin konnte gezeigt werden, dass auch eine terminale Sterilisation der Produkte Midazolam und GTN in kollabierbaren Tuben möglich ist ohne die Qualität des Produktes zu beeinträchtigen. Dies erlaubt es die Parenteralia/ parenteralen Lösungen, steril, stabil und zum direkten Einsatz in Spritzenpumpen bereitzustellen. Unter Parenteralia oder parenteralen Lösungen werden definitionsgemäß sterile Zubereitungen angesehen, die zur Injektion oder Infusion in den menschlichen Körper bestimmt sind.

Die in der vorliegenden Erfindung eingesetzten Tuben sind großvolumige etwa 50 ml umfassende Tuben, aufgebaut aus halbstarren Folien und bestehend aus einer Trägerfolie, die als Barriereschicht dient. Als Barriereschicht wird definitionsgemäß eine Schicht angesehen, die weniger Luft und Flüssigkeiten durchlässt als PE (Polyethylen) oder PP (Polypropylen). Als luft- und flüssigkeitsundurchlässige Barriereschicht wird beispielsweise PCTFE (Polychlortrifluorethylen) angenommen. Des Weiteren ist die Trägerfolie mit einem Innen- und Außenmantel aus einem thermoplastischen Material überzogen. Die bevorzugte Tube ist aus einer Trägerfolie bestehend aus PCTFE mit einem Innen- und Außenmantel aus PP aufgebaut. Der Tubenkopf besteht ebenfalls aus PP und weist einen weiblichen Luer-Lock Anschluss auf. Weiterhin ist der Tubenkopf durch eine Kunststofffolie versiegelt, die ebenfalls aus PP aufgebaut ist. Möglich ist ebenfalls eine Ausführung einer Tube, in der der Tubenkopf aus COC (Cyclo Olefin Copolymeren) besteht. Eine ähnliche Tube ist beispielsweise beschrieben in

Patentanmeldung WO 2012/079180 der Firma Hoffmann Neopac AG.

Die vorliegende Erfindung ermöglicht es so durch Eindrehen einer standardmäßigen 50 ml Spritze mit männlichem Luer Lock Anschluss die Kunststoffversiegelung zu durchbrechen und die fertige Infusionslösung ohne Kontaminationsrisiko und ohne Einsatz weiterer Kanülen oder anderer Hilfsmittel direkt in die Spritze aufzuziehen. Durch die Bereitstellung der Parenteralia als gebrauchsfertige 50 ml Lösungen entfallen jegliche weiteren Transfer- und Verdünnungs- oder Mischungsschritte.

Die flexible Ausgestaltung der eingesetzten Tube ermöglicht es zudem durch Kollabieren des Tubenkörpers die Lösung vollständig und ohne großen Kraftaufwand sowie ohne Druckausgleich durch Zuführen von Umgebungsluft in die 50 ml Spritze zu überführen.

Eine Ausführungsform der Erfindung beschreibt Lösungen parenteral zu verabreichender Wirkstoffe (auch parenterale Lösungen genannt) zum Einsatz in Dosierpumpen. Erfindungsgemäß werden die Lösungen lagerstabil und gebrauchsfertig zur direkten, nadelfreien Aufnahme in großvolumige Spritzen bereitgestellt.

In einer bevorzugten Ausgestaltung der vorliegenden Erfindung werden die oben beschriebenen parenteralen Lösungen zum Einsatz in Dosierpumpen in kollabierbaren Behältern bereitgestellt, die mit einem weiblichen Luer-Lock Anschluss ausgestattet sind. Mindestens eine Behälterwand der kollabierbaren Behälter enthält eine luft- und flüssigkeitsundurchlässige Barriereschicht.

In einer besonders bevorzugten Ausführungsform sind die zu verabreichenden Wirkstoffe, der wie oben beschriebenen parenteralen Lösungen, ausgewählt aus Atracurium, Cis-Atracurium, GTN oder Midazolam. Die Lösungen zum Einsatz in Dosierpumpen sind lagerstabil und gebrauchsfertig zur direkten, nadelfreien Aufnahme in Spritzen bereitgestellt.

In einer bevorzugten Ausgestaltung der vorliegenden Erfindung werden die oben beschriebenen parenteralen Lösungen zum Einsatz in Dosierpumpen in kollabierbaren Behältern bereitgestellt, die vorzugswiese Atracurium, Cis-Atracurium, GTN oder Midazolam enthalten. Die Behälter sind mit einem weiblichen Luer-Lock Anschluss ausgestattet und mindestens eine Behälterwand der kollabierbaren Behälter enthält eine luft- und flüssigkeitsundurchlässige Barriereschicht.

Eine Ausführungsform der Erfindung beschreibt Lösungen parenteral zu verabreichender Wirkstoffe zum Einsatz in Dosierpumpen, die in ihrem Behältnis terminal sterilisierbar sind. Unter einer terminalen Sterilisation wird eine thermische Sterilisation verstanden, die einen F0 Wert von ≥ 8 erreicht. Bevorzugt wird die terminale Sterilisation bei 121 °C durchgeführt. Erfindungsgemäß werden die Lösungen lagerstabil und gebrauchsfertig zur direkten, nadelfreien Aufnahme in großvolumige Spritzen bereitgestellt.

In einer weiteren bevorzugten Ausgestaltung der Erfindung sind die wie oben beschriebenen gebrauchsfertigen parenteralen Lösungen zum Einsatz in Dosierpumpen in einem kollabierbaren Behälter terminal sterilisierbar. Mindestens eine Behälterwand, der Behälter dieser Ausführungsform enthält eine luft- und feuchtigkeitsundurchlässige Barriereschicht. Der Behälter ist zudem mit einem weiblichen Luer Lock Anschluss ausgestattet.

Die Lösungen enthalten bevorzugt die Wirkstoffe Midazolam oder GTN.

Eine besonders bevorzugte Ausführungsform der Erfindung beschreibt Lösungen parenteral zu verabreichender Wirkstoffe zum Einsatz in Dosierpumpen, die ein Volumen von 50 ml umfassen. Erfindungsgemäß werden die Lösungen lagerstabil und gebrauchsfertig zur direkten, nadelfreien Aufnahme in großvolumige Spritzen bereitgestellt.

In einer bevorzugten Ausgestaltung der vorliegenden Erfindung werden die oben beschriebenen parenteralen Lösungen zum Einsatz in Dosierpumpen in kollabierbaren

Behältern bereitgestellt, wobei die Lösungen bevorzugt ein Volumen von 50 ml umfassen. Mindestens eine Behälterwand der Behälter dieser Ausführungsform enthält eine luft- und feuchtigkeitsundurchlässige Barriereschicht. Der Behälter ist zudem mit einem weiblichen Luer Lock Anschluss ausgestattet.

Die Lösungen enthalten bevorzugt die Wirkstoffe Midazolam, Cisatracurium, Atracurium oder GTN.

In einer besonders bevorzugten Ausführungsform werden die parenteral zu verabreichenden Wirkstoffe zum Einsatz in Dosierpumpen in einem kollabierbaren Behälter bereitgestellt, wobei der Behälter eine Tube darstellt. Erfindungsgemäß werden die Lösungen in der Tube lagerstabil und gebrauchsfertig zur direkten, nadelfreien Aufnahme in großvolumige Spritzen bereitgestellt.

In einer besonders bevorzugten Ausführungsform ist der kollabierbare Behälter zur Aufnahme der erfindungsgemäßen parenteralen Lösungen zur Verabreichung über Dosierpumpen eine Tube mit einem Volumen von 50-60 ml. Mindestens eine Behälterwand der erfindungsgemäßen Tube enthält eine luft- und feuchtigkeitsundurchlässige Barriereschicht. Die Tube ist zudem mit einem weiblichen Luer Lock Anschluss ausgestattet. Die Lösungen enthalten bevorzugt die Wirkstoffe Midazolam, Cisatracurium, Atracurium oder GTN. Besonders bevorzugt sind die Lösungen in der Tube terminal sterilisierbar.

Erfindungsgemäß wird eine kollabierbare Tube dazu verwendet, eine großvolumige Spritze, die zum Einsatz in Dosierpumpen geeignet ist, mit parenteral zu verabreichenden Wirkstoffen zu befüllen. Bevorzugt ist die Verwendung einer erfindungsgemäßen, kollabierbaren Tube, bei der mindestens eine Behälterwand eine luft- und feuchtigkeitsundurchlässige Barriereschicht enthält, wobei die Produkte lagerstabil sind.

Bevorzugt wird eine kollabierbare Tube, die besonders bevorzugt ein Volumen von etwa 50 ml umfasst, erfindungsgemäß dazu verwendet, eine großvolumige Spritze, die zum Einsatz in Dosierpumpen geeignet ist, zu befüllen. Mindestens eine Behälterwand der Tube enthält eine luft- und feuchtigkeitsundurchlässige Barriereschicht. Die Produkte sind lagerstabil.

Besonders bevorzugt ist die erfindungsgemäße Verwendung der kollabierbaren Tube, bei der mindestens eine Behälterwand eine luft- und feuchtigkeitsundurchlässige Barriereschicht enthält, zur Aufnahme von parenteral zu verabreichenden Wirkstoffen ausgewählt aus Atracurium, Cis-Atracurium, GTN oder Midazolam. Die Produkte sind dabei in der Tube lagerstabil. Die Tube umfasst bevorzugt ein Volumen von 50 ml.

In einem erfindungsgemäßen Verfahren wird eine großvolumige Spritze, die zum Einsatz in Dosierpumpen geeignet ist direkt über einen Luer Lock Anschluss aus einem kollabierbaren Behälter mit einer gebrauchsfertigen, parenteralen Lösung befüllt.

In einem bevorzugten Verfahren enthält der kollabierbare Behälter gebrauchsfertige, parenterale Lösungen, die direkt über einen Luer Lock Anschluss in die Spritze, die zum Einsatz in Dosierpumpen geeignet ist, aufgezogen werden, enthaltend einen der Wirkstoffe Atracurium, Cis-Atracurium, GTN oder Midazolam.

In einem besonders bevorzugten Verfahren ist der kollabierbare Behälter für gebrauchsfertige, parenterale Lösungen, die direkt über einen Luer Lock Anschluss in die Spritze aufgezogen werden, die zum Einsatz in Dosierpumpen geeignet ist, eine Tube.

In einem besonders bevorzugten Verfahren ist der kollabierbare Behälter für gebrauchsfertige, parenterale Lösungen, die direkt über einen Luer Lock Anschluss in die Spritze aufgezogen werden, die zum Einsatz in Dosierpumpen geeignet ist, eine Tube. Die erfindungsgemäßen gebrauchsfertigen, parenteralen Lösungen sind in der Tube lagerstabil.

In einem weiteren besonders bevorzugten Verfahren enthält die Tube zum Befüllen der Spritze, die zum Einsatz in Dosierpumpen geeignet ist einen der Wirkstoffe ausgewählt aus Atracurium, Cis-Atracurium, GTN oder Midazolam.

In einem weiteren besonders bevorzugten Verfahren enthält die Tube zum Befüllen der Spritze, die zum Einsatz in Dosierpumpen geeignet ist einen der Wirkstoffe ausgewählt aus Atracurium, Cis-Atracurium, GTN oder Midazolam. Die erfindungsgemäßen parenteralen Lösungen der bevorzugten Wirkstoffe sind in der Tube lagerstabil.

### Beispiele:

Die folgenden detaillierten Beispiele sind als rein beschreibende Ausführungsbeispiele dargestellt und sind in keiner Weise als Beschränkungen der vorliegenden Erfindung auszulegen.

### Beispiel 1: Midazolam Lösung für Spritzenpumpen:

Die folgende Lösung 2 mg/ml Midazolam in Wasser für Injektionszwecke wurde hergestellt und in Tuben (Trägerfolie Polychlortrifluorethylen (PCTFE), Ummantelung Polypropylen (PP), Kopf PP mit PP-Versiegelung) abgefüllt.

**Tabelle 1: Formulierung Midazolam 2 mg/ml**

| **Inhaltsstoff** | **Menge pro 50 ml** |
|---|---|
| Midazolam HCl | 111.2 mg |
| NaCl | 431.4 mg |
| 1 N HCl | 332.3 - 370 3 mg |
| Wfl | 49670.2 - 49707.2 mg |

Anschließend wurden die Tuben terminal sterilisiert (15 min., 121 °C) und unter Bedingungen der ICH Richtlinie Q1a für halbdurchlässige Behälter in Stabilitätskammern (Bedingungen: 40 °C, 25 % RH) eingelagert.

**Tabelle 2: Stabilitätsdaten Midazolam 2 mg/ml, Bedingungen: 40 °C ± 2 °C, 25 % ± 5 % RH**

| **Testparameter** | **Spezifikation** | **Ergebnisse** | | | |
|---|---|---|---|---|---|
| | | **T0** | **T15** | **T30** | **T60** |
| pH | 2.9 - 3.7 | 3.44 | 3.45 | 3.52 | 3.48 |
| Gehalt Midazolam UV [mg/ml] | 1.900 - 2.100 mg/m | 1.988 | 1.980 | 1.987 | 1.964 |

| Verunreinigungen (HPLC) [%] | | | | | |
|---|---|---|---|---|---|
| Dihydromidazolam | < 0.20 % | n.d. | n.d. | n.d. | 0.077 |
| Desfluoromidazolam | < 0.20 % | 0.010 | 0.012 | 0.014 | 0.110 |
| 6H-Midazolam | < 0.20 % | n.d. | n.d. | n.d. | 0.113 |
| O-Midazolam | < 0.20 % | n.d. | n.d. | n.d. | 0.034 |
| Lactam | < 0.20 % | 0.006 | 0.009 | 0.0010 | n.d. |
| Quinazolin | < 0.20 % | n.d. | n.d. | n.d. | n.d. |
| Unbekannte VU (single, max.) | < 0.20 % | RRT 0.14 - 0.053 | RRT 0.14 - 0.063 | RRT 0.14 - 0.046 | RRT 0.14 - 0.052 |
| | | RRT 0.76 - 0.023 | RRT 0.76 - 0.023 | RRT 0.76 - 0.026 | RRT 0.48 - 0.023 |
| | | | | | RRT 0.76 - 0.042 |
| Gesamte VU | < 0.50 % | 0.053 | 0.063 | 0.072 | 0.094 |

### Beispiel 2: GTN Lösung für Spritzenpumpen

Die folgende Lösung 1 mg/ml GTN in Wasser für Injektionszwecke wurde hergestellt und in Tuben (Trägerfolie PCTFE, Ummantelung PP, Kopf PP mit PP-Versiegelung) abgefüllt.

**Tabelle 3: Formulierung GTN 1 mg/ml**

| **Inhaltsstoff** | **Menge pro 50 ml** |
|---|---|
| GTN Glukose | 2550.0 mg |
| Glukose Monohydrat | 250.0 mg |
| 3 N HCl | 1.3 - 2.35 mg |
| Wfl | 48098.7 - 48097.7 mg |

Anschließend wurden die Tuben terminal sterilisiert (15 min., 121 °C) und unter Bedingungen der ICH Richtlinie Q1a für halbdurchlässige Behälter in Stabilitätskammern (Bedingungen: 25 °C, 40 % RH und 40 °C, 25 % RH) eingelagert.

**Tabelle 4: Stabilitätsdaten Glycerin Trinitrat 1 mg/ml, Bedingungen: 25 °C ± 2 °C, 40 % ± 5 % RH**

| **Testparameter** | **Spezifikation** | **Ergebnisse** | |
|---|---|---|---|
| | | **T0** | **T90** |
| pH | 3.0 - 4.0 | 3.53 | 3.54 |
| Gehalt GTN HPLC [%] | 95 - 105 % | 100 | 101.1 |
| 5-Hydroxymethylfurfural (UV) | ABS < 0.25 | 0.07 | 0.08 |

| Verunreinigungen (HPLC) [%] | | | |
|---|---|---|---|
| Glyceryl-1-mononitrat | < 0.5 % | 0.079 | 0.044 |
| Glyceryl-2-mononitrat | < 0.5 % | n.d. | n.d. |
| Glyceryl-1,2-dinitrat | < 2.0 % | 0.786 | 0.850 |
| Glyceryl-1,3-dinitrat | < 1.0 % | 0.290 | 0.322 |
| Unbekannte VU (single, max.) | < 1.0 % | RRT 0.05 - 0.031 | RRT 0.06 - 0.014 |
| | | RRT 0.17 - 0.240 | RRT 0.17 - 0.289 |
| Gesamte unbekannte VU | < 1.0 % | Σ0.271 | Σ0.346 |

**Tabelle 5: Stabilitätsdaten Glycerin Trinitrat 1 mg/ml, Bedingungen: 40 °C ± 2 °C, 25 % ± 5 % RH**

| **Testparameter** | **Spezifikation** | **Ergebnisse** | | | | |
|---|---|---|---|---|---|---|
| | | **T0** | **T15** | **T30** | **T60** | **T90** |
| pH | 3.0 - 4.0 | 3.53 | 3.54 | 3.51 | 3.54 | 3.49 |
| Gehalt GTN HPLC [%] | 95 - 105 % | 100 | 99.9 | 101.5 | 101.8 | 102.2 |
| 5-Hydroxymethylfurfural (UV) | < 0.25 | 0.07 | 0.08 | 0.09 | 0.11 | 0.13 |

| Verunreinigungen (HPLC) [%] | | | | | | |
|---|---|---|---|---|---|---|
| Glyceryl-1-mononitrat | < 0.5 % | 0.079 | 0.034 | 0.049 | 0.031 | 0.047 |
| Glyceryl-2-mononitrat | < 0.5 % | n.d. | 0.035 | n.d. | n.d. | n.d. |
| Glyceryl-1,2-dinitrat | < 2.0 % | 0.786 | 0.883 | 0.863 | 1.017 | 1.151 |
| Glyceryl-1,3-dinitrat | < 1.0 % | 0.290 | 0.328 | 0.325 | 0.349 | 0.409 |
| Unbekannte VU (single, max.) | < 1.0 % | RRT 0.17 - 0.240 | RRT 0.17 - 0.310 | RRT 0.17 - 0.328 | RRT 0.17 - 0.444 | RRT 0.17 - 0.504 |
| | | | | RRT 0.32 - 0.033 | RRT 0.32 - 0.021 | RRT 0.32 - 0.037 |
| Gesamte unbekannte VU | < 1.0 % | 0.271 | 0.310 | 0.563 | 0.475 | 0.566 |

### Beispiel 3: Atracurium 10 mg/ml Lösung

90 % der nötigen Menge Wasser für Injektionszwecke wurden mit Stickstoff bis zu einem Restsauerstoffgehalt unter 0.2 mg/ml begast. Anschließend wurde Atracurium Besilat zugegeben und gerührt bis der Feststoff gelöst war. Währenddessen wurde eine 0.1 %ige Lösung Benzolsulfonsäure hergestellt und für 15 min. gerührt. Durch Zusammenführen der beiden Lösungen wurde dann der pH Wert auf 3.4 eingestellt und weiterhin mit Stickstoff begast. Anschließend wurde mit Wfl auf das Endvolumen aufgefüllt und für weitere 30 min. unter Begasung gerührt. Anschließend wurde die Lösung durch einen Fluorodyne-Filter filtriert und unter aseptischen Bedingungen in Tuben (Trägerfolie PCTFE, Ummantelung PP, Kopf PP mit PP-Versiegelung) abgefüllt. Die produzierten Tuben wurden unter Bedingungen der ICH Richtlinie Q1a für halbdurchlässige Behälter in Stabilitätskammern (Bedingungen: 5 °C, und 25 °C, 40 % RH) eingelagert.

Bei Atracurium handelt es sich um ein Kühlprodukt, dass bei 2 °C - 5 °C gelagert werden muss. Eine terminale Sterilisation ist auch im Glasvial nicht möglich.

**Tabelle 6: Stabilitätsdaten Atracurium 10 mg/ml, Bedingungen: 5 °C ± 3 °C**

| **Testparameter** | **Spezifikation** | **Ergebnisse** | |
|---|---|---|---|
| | | **T0** | **T90** |
| pH | 3.00 - 3.65 | 3.52 | 3.43 |
| Gehalt Atracurium Besilat [mg/ml] | 9.0 - 12.0 mg/ml | 11.39 | 11.06 |

| Verunreinigungen (HPLC) [%] | | | |
|---|---|---|---|
| 7115 | < 6.5 % | 0.114 | 0.665 |
| 7140 | < 2.0 % | 0.200 | 0.268 |
| 7113 trans + cis | < 7.0 % | trans - 0.092 cis - 0.270 | trans - 0.262 cis - 0.789 |
| 7114 trans + cis | < 5.0 % | trans-0.121 cis - 0.713 | trans - 0.526 cis - 0.834 |
| Unbekannte VU (single) | < 0.2 % | RRT 1.19 - 0.088 | RRT 0.87 - 0.059 RRT 1.19-0.115 |
| Gesamt VU | < 17.5 % | Σ0.288 | Σ1.896 |

**Tabelle 7: Stabilitätsdaten Atracurium 10 mg/ml, Bedingungen: 25 °C, 40 % RH**

| **Testparameter** | **Spezifikation** | **Ergebnisse** | | | | |
|---|---|---|---|---|---|---|
| | | **T0** | **T15** | **T30** | **T60** | **T90** |
| pH | 3.00 - 3.65 | 3.52 | 3.46 | 3.42 | 3.26 | 3.21 |
| Gehalt Atracurium Besilat [mg/ml] | 9.0 - 12.0 mg/ml | 11.39 | 11.10 | 10.98 | 10.56 | 10.02 |

| Verunreinigungen | | | | | | |
|---|---|---|---|---|---|---|
| (HPLC) [%] | < 6.5 % | 0.114 | 0.756 | 1.368 | 2.492 | 3.802 |
| 7115 | < 2.0 % | 0.200 | 0.442 | 0.651 | 0.988 | 1.261 |
| 7140 | < 7.0 % | t - 0.092 | t - 0.278 | t - 0.434 | t - 0.733 | t - 1.097 |
| 7113 trans + cis | | c - 0.270 | c - 0.865 | c - 1.361 | c - 2.403 | c - 3.444 |
| | < 5.0 % | t - 0.121 | t - 0.516 | t - 0.654 | t - 1.072 | t - 0.673 |
| 7114 trans + cis | | c - 0.713 | c - 1.530 | c - 2.223 | c - 3.342 | c - 4.098 |
| Unbekannte VU (single) | < 0.2 % | RRT 1.19-0.088 | RRT 1.19 < 0.05 | RRT 1.19 n.d. | RRT 1.19 n.d. | RRT 1.19 < 0.05 |
| Gesamt VU | < 17.5 % | Σ0.288 | Σ3.593 | Σ6.691 | Σ11.030 | Σ13.702 |

## Patentansprüche

1. Lösungen parenteral zu verabreichender Wirkstoffe zum Einsatz in Dosierpumpen, **dadurch gekennzeichnet, dass** die Lösungen lagerstabil und gebrauchsfertig zur direkten, nadelfreien Aufnahme in großvolumige Spritzen bereitgestellt werden.

2. Parenterale Lösungen nach Anspruch 1, **dadurch gekennzeichnet, dass** die Lösungen in kollabierbaren Behältern bereitgestellt werden, die mit einem weiblichen Luer Lock- Anschluss ausgestattet sind, wobei mindestens eine Behälterwand eine luft- und flüssigkeitsundurchlässige Barriereschicht enthält.

3. Parenterale Lösungen nach Anspruch 1 oder 2, **dadurch gekennzeichnet, dass** die zu verabreichenden Wirkstoffe ausgewählt sind aus Atracurium, Cis-Atracurium, GTN oder Midazolam.

4. Parenterale Lösungen nach einem der Ansprüche 1-3, **dadurch gekennzeichnet, dass** die Lösungen im Behälter terminal sterilisierbar sind.

5. Parenterale Lösungen nach einem der Ansprüche 1-4, die ein Volumen von 50 ml umfassen.

6. Parenterale Lösungen nach einem der Ansprüche 1-5, wobei der kollabierbare Behälter eine Tube darstellt.

7. Verwendung einer großvolumigen, kollabierbaren Tube zur Aufnahme von parenteral zu verabreichenden Wirkstoffen, wobei mindestens eine Behälterwand eine luft - und feuchtigkeitsundurchlässige Barriereschicht enthält, und die Produkte lagerstabil sind.

8. Verwendung einer Tube nach Anspruch 7, **dadurch gekennzeichnet, dass** die Tube ein Volumen von 50 ml umfasst.

9. Verwendung einer Tube nach Anspruch 7 oder 8, wobei die aufzunehmenden, parenteral zu verabreichenden Wirkstoffe ausgewählt sind aus Atracurium, Cis-Atracurium, GTN oder Midazolam.

10. Verfahren zur Befüllung großvolumiger Spritzen, die für den Einsatz in Dosierpumpen geeignet sind, **dadurch gekennzeichnet, dass** die parenteralen Lösungen gebrauchsfertig aus kollabierbaren Behältern direkt über einen Luer-Lock Anschluss in die Spritze aufgezogen werden.

11. Verfahren nach Anspruch 10, **dadurch gekennzeichnet, dass** die parenteralen Lösungen ausgewählt sind aus Lösungen enthaltend einen der Wirkstoffe Atracurium, Cis-Atracurium, GTN oder Midazolam.

12. Verfahren nach Anspruch 10 oder 11, **dadurch gekennzeichnet, dass** der kollabierbare Behälter eine Tube ist.
